# EUROPEAN PATENT SPECIFICATION

(11) **EP 1 401 507 B1**
(45) Date of publication and mention of the grant of the patent: **07.01.2009**
(21) Application number: 02726875.4
(22) Date of filing: 16.05.2002
(51) Int. Cl.: A61K 51/00, A61M 36/14, A61K 51/04, A61K 51/08, A61K 51/10

(54) **STABILIZATION OF RADIONUCLIDE-CONTAINING COMPOSITIONS**
STABILISIERUNG VON RADIONUKLID ENTHALTENDEN ZUSAMMENSETZUNGEN
STABILISATION DE COMPOSITIONS CONTENANT UN RADIONUCLEIDE

(30) Priority: 16.05.2001 US 855542
(43) Date of publication of application: 31.03.2004
(73) Proprietor: Bayer Schering Pharma Aktiengesellschaft, 13353 Berlin (DE)
(72) Inventor: MANCHANDA, Rajesh, Brookline, MA 02445 (US)
(74) Representative: Ziebig, Marlene
(86) International application number: PCT/US2002/015360
(87) International publication number: WO 2002/092005

(56) References cited:
- US-A- 5 262 175
- US-A- 5 807 537
- US-B1- 6 174 513
- MILLAR A M: "A routine method for using sodium iodide to stabilize sodium pertechnetate [99Tcm] dispensed for the preparation of 99Tcm-exametazime." NUCLEAR MEDICINE COMMUNICATIONS. MAY 1992, vol. 13, no. 5, May 1992 (1992-05), pages 306-311, XP008040301 ISSN: 0143-3636

## Description

The invention is as described below and includes methods for stabilizing radionuclide-containing compositions against degradation caused by free radicals generated from the radionuclide or other forms of radiolysis. The invention is also directed to compositions associated with these methods. Iodide ions stabilize the radionuclide-containing compositions by acting as scavengers to these generated free radicals, thus, preventing or lessening degradation therefrom or from other forms of radiolysis. Among the preferred radionuclide-containing compositions to be stabilized are compositions containing a targeting agent together with a radionuclide, whereby the targeting agent is associated with the radionuclide, preferably by being linked to a complexing agent which is capable of completing the radionuclide, for example, such as a diagnostic agent having as a targeting moiety a specific binding peptide, oligonucleotide, antibody or small organic targeting group linked to a metal ion-complexing moiety which is complexed with a radionuclide, such as technetium-99m (Tc-99m). Also included in the invention are compositions of radionuclides, radionuclide-containing compounds or complexes with iodide or an iodide ion-providing component; compositions of compounds or complexing agents that are associated with a radionuclide with iodide or an iodide ion-providing component; and kits containing any combination of radionuclides, targeting agents, complexing agents or compounds which are associated with or will be associated with radionuclides and iodide or iodide-providing components.

### Background of the Invention

Compounds, compositions and complexes containing radionuclides have been known for diagnostic and therapeutic applications. Among such embodiments are reagents having one or more components for binding a radionuclide, such as technetium-99m ("Tc-99m"), and a component for targeting the reagent to a specific site in the body, such as a mammalian body, particularly human. The reagents can be targeted to specific sites and the radionuclide used to carry out scintigraphic inzaging for diagnosis of the site. Therapeutic applications from such targeting are possible as well. Examples of such reagents are described in U.S. patents 5,783,170; 5,807,537; 5,814,297; and 5,866,097. Particularly disclosed as reagents are complexes of the radionuclide with a complexing group which complexes the radionuclide and which is covalently bonded to a specific binding peptide for targeting the complex. Such complexes are useful for a variety of diagnostic and therapeutic methods, such as discussed in the above-cited U.S. patents.

A drawback of radionuclides and compositions er complexes containing them is degradation over time through radiolysis of the complexed radionuclide. Thus, after formation of the complex, the radiochemical purity ("RCP" in % indicating the extent of stability of the moiety containing the radionuclide) will diminish and hinder the effectiveness of the reagent. For example, U.S. Patent No. 5,262,175 discloses that a certain Tc-99m labeled complex made through the Ceretec kit has an in-vitro shelf life an the order of only 30 minutes. This patent discloses stabilization of radiopharmaceutical complex compositions with a weak oxidizing agent. The preferred weak oxidizing agent is sodium hypochlorite but several others are listed, including iodine.

Millar A.M. describes in NUCLEAR MEDICINE COMMUNICATIONS. MAY 1992, vol. 13, no. 5, May 1992 (1992-05), pages 306-311, a method using iodide to stabilize sodium pertechnetate ⁹⁹Tc^{m} for the preparation of ⁹⁹Tc^{m}-exametazime.

### Summary of the Invention

The invention includes methods for stabilizing radionuclide-containing compositions against degradation caused by free radicals. The invention is also directed to compositions associated with these methods. Such free radicals are generally derived from the radionuclide due to formation of hydrated electrons. It has been discovered that iodide ions stabilize the radionuclide-containing compositions by acting as scavengers to the generated free radicals, thus, preventing or lessening degradation caused by such free radicals.

Among the preferred radionuclide-containing compositions to be stabilized are complexes of a complexing agent with a radionuclide complexed therewith, such as a diagnostic agent having a specific binding peptide linked to a mental ion-complexing moiety which is complexed with a radionuclide, such as technetium-99m (Tc-99m). In addition to methods for stabilizing radionuclide-containing compositions, also included in the invention are compositions of radionuclide-containing compounds or complexes with iodide or an iodide ion-providing component, compositions of compounds or complexing agents that are associated with a radionuclide and kits containing any combination of radionuclides, radionuclide generators, complexing agents or compounds which are associated with or will be associated with radionuclides and iodide or iodide-providing components. Further aspects of the invention are taught to one of ordinary skill in the art from the disclosure as a whole.

As the compositions which are stabilized by the iodide according to the invention are included any compositions which contain a radionuclide, particularly these which are susceptible to degradation, hence a reduction in RCP. Though not limited thereto, the invention is particularly applicable to stabilizing compositions having a radionuclide associated (by covalent binding, other binding forces or merely in admixture) with a targeting agent The targeting agent is a compound or moiety that targets or directs the radionuclide to a specific site in a biological system. Preferably the targeting moiety is a peptide, oligonucleotide or antibody, particularly one which has specificity to target the complex to a specific site in a biological system. Smaller organic molecules effective for targeting certain sites in a biological system can also be used as the targeting agents with the invention. Such targeting agents are known in the art and are described in U.S. Patent Nos. 5,783,170; 5,807,537; 5,814,297; 5,866,097; and 5,262,175 mentioned above and elsewhere, see, e.g., 5,736,122; 5,849,260; 5,879,658; 5,888,474; 5,716,596; 5,814,298; 5,820,845; 5,552,525; 5,561,220; 5,714,579; and 5,711,931. Methods for preparing them are discussed in those patents and/or are known in the art. Referred as targeting agents are peptides comprising from 4 to 100 amino acids or oligonucleotides with 4-100 nucleotides or antibodies or peptidomimetics; these, preferably being covalently linked to a complexing group which binds the radionuclide.

In another preferred, but non-limiting, embodiment the radionuclide is contained in the composition to be stabilized at least partially complexed by a complexing moiety. Examples of complexing moieties and compositions containing complexed radionuclides which can be stabilized according to the invention include those described in each of U.S. Patent Nos. 5,783,170; 5,807,537; 5,814,297; 5,866,097; and 5,262,175 discussed above. One preferred type of complexing moiety is a thiol group-containing moiety such as of the following formula:

A-CZ(B)-[C(R¹R²)]ₙ-X

wherein A is H, HOOC, H₂NOC, (peptide, oligonucleotide, or antibody)-NHOC, (peptide, oligonucleotide, or antibody)-OOC or R⁴; B is H, SH or -NHR³, -N(R³)-(peptide, oligonucleotide, or antibody) or R⁴; X is SH or -NHR³, -N(R³)-(peptide) or R⁴; R¹, R², R³ and R⁴ are independently H or straight or branched chain or cyclic lower alkyl; n is 0, 1 or 2; provided that: (a) where B is -NHR³ or -N(R³)-(peptide, oligonucleotide, or antibody), X is SH and n is 1 or 2; (b) where X is -NHR³ or -N(R³)-(peptide, oligonucleotide, or antibody), B is SH and n is 1 or 2; (c) where B is H or R⁴, A is HOOC, H₂NOC, (peptide, oligonucleotide, or antibody)-NHOC, (peptide, oligonucleotide, or antibody)-OOC, X is SH and n is 0 or 1; (d) where A is H or R⁴, then, where B is SH, X is -NHR³ or -N(R³)-(peptide, oligonucleotide, or antibody) and where X is SH, B is -NHR³ or -N(R³)-(peptide, oligonucleotide, or antibody); (e) where X is H or R⁴, A is HOOC, H₂NOC, (peptide)-NHOC, (peptide, oligonucleotide, or antibody)-OOC and B is SH; (f) where Z is methyl, X is methyl, A is HOOC, H₂NOC, (peptide, oligonucleotide, or antibody)-NHOC, (peptide, oligonucleotide, or antibody)-OOC and B is SH and n is 0; and (g) where Z is SH and X is SH, n is not 0; and wherein the thiol moiety is in the reduced form and the complexing group is preferably capable of being covalently linked to a peptide, oligonucleotide, or antibody.

In a preferred embodiment, compositions having a radionuclide and a somatostatin receptor ("SSTR") binding peptide, such as depreotide or P2045, are stabilized by iodide. Preferably the SSTR binding peptide is linked to a complexing agent which at least partially complexes the radionuclide.

The radionuclide which is stabilized may be selected from any known radionuclide. The invention is particularly applicable, however, to stabilizing compositions containing Tc or Re radionuclides, particularly Tc-99m and Re-188. Other possible radionuclide-containing compositions which can be stabilized by the invention include those having Re-186, Ga-67, In-111, I-123, I-125, I-131 and Yb-169 radionuclides. The invention could also be applied to stabilize any radioisotope, such as H-3, C-14, N-15, F-18, P-32, P-33 or Y-90.

The iodide ion used for stabilization according to the invention may be derived from any known source. Particularly useful are iodide salts which provide the iodide ion in solution and which are biocompatible. Most preferred are alkali metal iodide salts, particularly KI and NaI. It is also possible to use reagents which generate iodide ions under the conditions in which the radionuclide-containing composition is provided; for example, ammonium iodides, such as Bu₄N⁺I- and NH₄⁺I-.

An amount of iodide-providing compound is added sufficient to provide stabilization of the radionuclide, radionuclide-containing composition and/or complexed radionuclide such that, for example, the radiochemical purity, RCP, is 90% or greater for at least the half-life of the radionuclide being stabilized, e.g., at least 6 hours for Tc-99m. Thus, the invention is also directed to compositions which contain a radionuclide-containing reagent and iodide ion or reagent which generates iodide ion. Further, the invention includes compositions containing a targeting agent, optionally having a complexing moiety linked thereto, before being associated or complexed with the radionuclide, and the iodide ion or reagent which generates it in the above-discussed sufficient amounts.

The iodide ion or compound which releases or generates such ion may be added to the radionuclide-containing composition any time before, during or after associating or complexing of the radionuclide with the targeting and/or complexing agent. It is preferred that the iodide ion be provided before association or complexing of the radionuclide in order to maximize the stabilizing effect. Thus, the iodide or iodide generating compound can be added to the targeting agent optionally having a complexing moiety before it is associated or complexed with the radionuclide. Thus, also included in the invention are kits useful for making the above-described compositions. For example, useful kits may include one compartment carrying the compositions of targeting agent, optionally with complexing moiety, with the iodide ions or iodide-providing compound and another compartment for carrying the radionuclide or ingredients for generating the radionuclide. In another embodiment the kit may contain the targeting agent, iodide providing compound, and radionuclide generating ingredients each separately.

According to the invention, radionuclide-containing compositions are stabilized sufficiently to significantly increase the shelf life of the compositions. For example, the RCP of such compositions may be maintained at the desired level of 90% or higher for up to a time equal to the half-life of the radionuclide after formation of the composition. This significantly enhances the usefulness of these reagents: The stabilizing effect of the iodide ions is even demonstrated when nitrate ions, which generally lead to increased degradation of radionuclide compositions or complexes, are present. This is of particular advantage because many compositions or kits used to generate radionuclides, such as CIS eluate, contain an oxidant, such as nitrates. By use of the iodide ions, it is possible to obtain good stabilization even when used together with these oxidant-containing radionuclide solutions.

The stabilized radionuclide-containing compositions of the invention are useful for diagnostic and therapeutic methods, particularly for scintigraphic imaging of a particular tissue of the biological system which is targeted by a peptide, particularly in mammalian systems, most particularly in human systems. The compositions can be selected, for example, for targeting and thus imaging of organs, such as the heart, the brain, blood vessels (e.g. arteries and veins) and tumors associated with diseases, for example, gastrointestinal tumors, myelomas, small cell lung carcinoma and other APUDomas, endocrine tumors such as medullary thyroid carcinomas and pituitary tumors, brain tumors such as meningiomas and astrocytomas, and tumors of the prostate, breast, colon and ovaries, for example. Methods for conducting the imaging with administration of a radionuclide reagent are conventionally known in the art. An advantage particular to the claimed invention is that the iodide stabilizing agent can be provided by reagents, such as potassium iodide, which are well tolerated by biological systems, particularly humans.

The entire disclosure of all applications, patents and publications, cited above and below is hereby incorporated by reference.

### Examples

In the foregoing and in the following examples, all temperatures are set forth uncorrected in degrees Celsius; and, unless otherwise indicated, all parts and percentages are by weight

### Example 1

CIS eluate, a Tc-99m generator, was mimicked by adding an oxidant (sodium nitrate, in this case) to Syncor (DuPont) eluate. NeoTect is a kit to provide a peptide-linked complexing agent called Tc 99m depreotide. The stability of Tc 99m depreotide (NeoTect Lot No. 51001503) with and without nitrate in the presence of potassium iodide (KI) was carried out to 9 hours post-reconstitution. The results of the RCP studies (at 9 h) are summarized below:

**Table 1: RCP of NeoTect at 9 hours post-reconstitution**

| **Sample** | **RCP %** |
|---|---|
| Control (1503 +Nitrate) | 48.1 |
| | |
| 1503 + 1 mg KI | 94.4 |
| 1503 + 1 mg KI | 88.5 |
| | |
| 1503 + nitrate + 1 mg KI | 78.8 |
| 1503 + nitrate + 5 mg KI | 94.3 |
| 1503 + nitrate + 10 mg KI | 92.6 |

KI affords stability to Tc-99m depreotide even when an oxidant (i.e. nitrate) is present in the eluate. Potassium iodide is well tolerated in humans unlike other stabilizing agents such as methionine or trolox. Methods for determining the RCP value are known in the art such as described in Cancer Res. (1998), May 1, 58(9):1850-1859, and J. Nucl. Med. (1996), June, 37(6):1016-1022.

### Example 2

Further examples were conducted to show the stabilizing effect of the iodide ion for other complexes under varying conditions and with varying amounts and sources (KI and NaI) of iodide ion. The results are shown in the following tables.

**Table 1: Single Vial - Iodide was added to the composition containing the targeting agent (peptide) and formulated as a single vial kit. The kit was reconstituted with Tc 99m to produce Tc 99m complexed to the targeting agent**

| **Targeting Agent** | **Iodide** | **Amount** | **%RCP** | **Time** |
|---|---|---|---|---|
| Depreotide | KI | 4 mg | 89% | 5 h |
| Depreotide | KI | 5 mg | 93% | 6 h |
| Depreotide | KI | 6 mg | 95% | 5 h |
| Depreotide | NaI | 8 mg | 91% | 6 h |
| Depreotide | NaI | 10 mg | 93% | 5 h |
| P2045 | NaI | 5 mg | 95% | 8 h |

**Table 2: 2-Vials - Iodide was added to a formulated kit that contained the targeting agent (peptide) followed by the addition of the radionuclide (Tc-99m) to produce Tc 99m complexed to the targeting agent.**

| **Targeting Agent** | **Iodide** | **Amount** | **%RCP** | **Time** |
|---|---|---|---|---|
| Depreotide | KI | 10 mg | 95% | 6 h |
| Depreotide | KI | 4 mg | 94% | 5 h |
| Depreotide | KI | 6 mg | 94% | 5 h |

It is evident from the tables above that the various amounts of iodide ions added either as part of a formulated kit with the targeting agent (single vial) or added to a formulated targeting agent prior to the addition of the radionuclide (2-vial), afford stabilization of the composition. The RCP of the compositions containing iodide remains high after addition of the radionuclide.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. A composition comprising:
- a radionuclide, optionally as part of a compound or complex, which is associated with a targeting agent, and
- iodide ions or a compound which releases or generates iodide ions, where the iodide ions aid in stabilizing the composition against degradation thus maintaining high radiochemical purity of the composition.

2. The composition of claim 1, wherein the iodide ions are provided by an iodide salt in the composition.

3. The composition of claim 1, wherein the iodide ions are provided by an alkali metal iodide salt in the composition.

4. The composition of claim 1, wherein the targeting agent is a peptide, oligonucleotide, antibody, peptidomimetic or small organic compound which has specific binding affinity targeting it to at least one tissue of a biological system.

5. The composition of claim 1, wherein the targeting agent is associated with the radionuclide by being bonded to a complexing moiety which complexes the radionuclide.

6. The composition of claim 5, wherein the targeting agent bonded to a complexing moiety is represented by the formula:
A--CZ(B)--[C(R¹R²)]ₙ-X
wherein A is H, HOOC, H₂NOC, (peptide, oligonucleotide, antibody or small organic compound)-NHOC,(peptide, oligonucleotide, antibody or small organic compound)-OOC or R⁴; B is H, SH or --NHR³,--N(R³)-(peptide, oligonucleotide, antibody or small organic compound) or R⁴; X is SH or -NHR³, -N(R³)-(peptide, oligonucleotide, antibody or small organic compound) or R⁴; R¹, R², R³ and R⁴ are independently H or straight or branched chain or cyclic lower alkyl; n is 0, 1 or 2; provided that: (a) where B is -NHR³ or -N(R³)-(peptide, oligonucleotide, antibody or small organic compound), X is SH and n is 1 or 2; (b) where X is -NHR³ or -N(R³)-(peptide, oligonucleotide, antibody or small organic compound), B is SH and n is 1 or 2; (c)where B is H or R⁴,A is HOOC, H₂NOC, (peptide, oligonucleotide, antibody or small organic compound)-NHOC, (peptide, oligonucleotide, antibody or small organic compound)-OOC, X is SH and n is 0 or 1; (d) where A is H or R⁴, then, where B is SH, X is -NHR³ or -N(R³)-(peptide, oligonucleotide, antibody or small organic compound) and where X is SH, B is -NHR³ or -N(R³)-(peptide, oligonucleotide, antibody or small organic compound); (e) where X is H or R⁴; A is HOOC, H₂NOC, (peptide, oligonucleotide, antibody or small organic compound)-NHOC, (peptide, oligonucleotide, antibody or small organic compound)-OOC and B is SH; (f) where Z is methyl, X is methyl, A is HOOC, H₂NOC, (peptide, oligonucleotide, antibody or small organic compound)-NHOC, (peptide, oligonucleotide, antibody or small organic compound)-OOC and B is SH and n is 0; and (g) where Z is SH and X is SH, n is not 0; and wherein the thiol moiety is in the reduced form and the complexing group is capable of being covalently linked to the peptide, oligonucleotide, antibody or small organic compound.

7. The composition of claim 4, wherein the targeting agent is a somatostatin receptor binding peptide.

8. The composition of claim 7, wherein the somatostatin receptor binding peptide is depreotide or P2045.

9. The composition of claim 1, wherein the radionuclide is Tc-99m.

10. A method for stabilizing a composition comprising a radionuclide associated with a targeting agent to prevent or lessen the occurrence of the radionuclide degrading, the method comprising providing iodide ions in the composition.

11. The method of claim 10, wherein the iodide ions are provided by an iodide salt in the composition.

12. The method of claim 10, wherein the iodide ions are provided by an alkali metal iodide salt in the composition.

13. The method of claim 10, wherein the targeting agent is a peptide, oligonucleotide, antibody, peptidomimetic or small organic compound which has specific binding affinity targeting it to at least one tissue of a biological system.

14. The method of claim 10, wherein the targeting agent is associated with the radionuclide by being bonded to a complexing moiety which complexes the radionuclide.

15. The method of claim 14, wherein the targeting agent bonded to a complexing moiety is represented by the formula:
A--CZ(B)-[C(R¹R²)]ₙ--X
wherein A is H, HOOC, H₂NOC, (peptide, oligonucleotide, antibody or small organic compound)-NHOC,(peptide, oligonucleotide, antibody or small organic compound)-OOC or R⁴; B is H, SH or --NHR³,--N(R³)-(peptide, oligonucleotide, antibody or small organic compound) or R⁴; X is SH or -NHR³, -N(R³)-(peptide, oligonucleotide, antibody or small organic compound) or R⁴; R¹, R², R³ and R⁴ are independently H or straight or branched chain or cyclic lower alkyl; n is 0, 1 or 2; provided that: (a) where B is -NHR³ or -N(R³)-(peptide, oligonucleotide, antibody or small organic compound), X is SH and n is 1 or 2; (b) where X is -NHR³ or -N(R³)-(peptide, oligonucleotide, antibody or small organic compound), B is SH and n is 1 or 2; (c)where B is H or R⁴,A is HOOC, H₂NOC, (peptide, oligonucleotide, antibody or small organic compound)-NHOC, (peptide, oligonucleotide, antibody or small organic compound)-OOC, X is SH and n is 0 or 1; (d) where A is H or R⁴, then, where B is SH, X is -NHR³ or -N(R³)-(peptide, oligonucleotide, antibody or small organic compound) and where X is SH, B is -NHR³ or --N(R³)-(peptide, oligonucleotide, antibody or small organic compound); (e) where X is H or R⁴; A is HOOC, H₂NOC, (peptide, oligonucleotide, antibody or small organic compound)-NHOC, (peptide, oligonucleotide, antibody or small organic compound)-OOC and B is SH; (f) where Z is methyl, X is methyl, A is HOOC, H₂NOC, (peptide, oligonucleotide, antibody or small organic compound)-NHOC, (peptide, oligonucleotide, antibody or small organic compound)-OOC and B is SH and n is 0; and (g) where Z is SH and X is SH, n is not 0; and wherein the thiol moiety is in the reduced form and the complexing group is capable of being covalently linked to the peptide, oligonucleotide, antibody or small organic compound.

16. The method of claim 10, wherein the targeting agent is a somatostatin receptor binding peptide.

17. The method of claim 16, wherein the somatostatin receptor binding peptide is depreotide or P2045.

18. The method of claim 10, wherein the radionuclide is Tc-99m.

19. The method of claim 13, wherein the biological system is a mammalian body.

20. The method of claim 19, further comprising administering the complex to a mammalian body and conducting scintigraphic imaging of the mammalian body.

21. A kit comprising:
(a) a targeting agent associated with a radionuclide,
(b) iodide ions or a compound which releases or generates iodide ions, which iodide ions prevent or lessen degradation of the radionuclide due to radiolysis or free ions, and
(c) components for generating a radionuclide associated with the targeting agent,
wherein the kit has two or three compartments, (c) is contained in a separate compartment from (a) or (b) and (a) and (b) may be in the same or different compartments.

22. The kit of claim 21, wherein the iodide ions are provided by an iodide salt.

23. The kit of claim 21, wherein the iodide ions are provided by an alkali metal iodide salt.

24. The kit of claim 21, wherein the targeting agent is a peptide, oligonucleotide, antibody, peptidomimetic or small organic compound which has specific binding affinity targeting it to at least one tissue of a biological system.

25. The kit of claim 21, wherein the targeting agent is capable of being associated with the radionuclide by being capable of being bonded to a complexing moiety which complexes the radionuclide.

26. The kit of claim 25, wherein the targeting agent bonded to a complexing moiety is represented by the formula:
A--CZ(B)--[C(R¹R²)]ₙ--X
wherein A is H, HOOC, H₂NOC, (peptide, oligonucleotide, antibody or small organic compound)-NHOC,(peptide, oligonucleotide, antibody or small organic compound)-OOC or R⁴; B is H, SH or --NHR³,--N(R³)-(peptide, oligonucleotide, antibody or small organic compound) or R⁴; X is SH or -NHR³, -N(R³)-(peptide, oligonucleotide, antibody or small organic compound) or R⁴; R¹, R², R³ and R⁴ are independently H or straight or branched chain or cyclic lower alkyl; n is 0, 1 or 2; provided that: (a) where B is -NHR³ or -N(R³)-(peptide, oligonucleotide, antibody or small organic compound), X is SH and n is 1 or 2; (b) where X is --NHR³ or -N(R³)-(peptide, oligonucleotide, antibody or small organic compound), B is SH and n is 1 or 2; (c)where B is H or R⁴,A is HOOC, H₂NOC, (peptide, oligonucleotide, antibody or small organic compound)-NHOC, (peptide, oligonucleotide, antibody or small organic compound)-OOC, X is SH and n is 0 or 1; (d) where A is H or R⁴, then, where B is SH, X is -NHR³ or -N(R³)-(peptide, oligonucleotide, antibody or small organic compound) and where X is SH, B is -NHR³ or --N(R³)-(peptide, oligonucleotide, antibody or small organic compound); (e) where X is H or R⁴; A is HOOC, H₂NOC, (peptide, oligonucleotide, antibody or small organic compound)-NHOC, (peptide, oligonucleotide, antibody or small organic compound)-OOC and B is SH; (f) where Z is methyl, X is methyl, A is HOOC, H₂NOC, (peptide, oligonucleotide, antibody or small organic compound)-NHOC, (peptide, oligonucleotide, antibody or small organic compound)-OOC and B is SH and n is 0; and (g) where Z is SH and X is SH, n is not 0; and wherein the thiol moiety is in the reduced form and the complexing group is capable of being covalently linked to the peptide, oligonucleotide, antibody or small organic compound.

27. The kit of claim 21, wherein the targeting agent is a somatostatin receptor binding peptide.

28. The kit of claim 27, wherein the somatostatin receptor binding peptide is depreotide or P2045.

29. The kit of claim 21, wherein the radionuclide is Tc-99m.

## Patentansprüche

1. Zusammensetzung, umfassend:
- ein Radionuklid, gegebenenfalls als Teil einer Verbindung oder eines Komplexes, das mit einem zielgerichteten Mittel verknüpft ist, und
- lodid-lonen oder eine Verbindung, die Iodid-Ionen freisetzt oder erzeugt, wobei die Iodid-Ionen zur Stabilisierung der Zusammensetzung gegen Abbau beitragen, so dass hohe radiochemische Reinheit der Zusammensetzung aufrechterhalten wird.

2. Zusammensetzung nach Anspruch 1, wobei die Iodid-Ionen mit Hilfe eines Iodid-Salzes in der Zusammensetzung bereitgestellt werden.

3. Zusammensetzung nach Anspruch 1, wobei die Iodid-Ionen mit Hilfe eines Alkalimetalliodid-Salzes in der Zusammensetzung bereitgestellt werden.

4. Zusammensetzung nach Anspruch 1, wobei das zielgerichtete Mittel ein Peptid, Oligonucleotid, Antikörper, eine peptidomimetische oder kleine organische Verbindung ist, die spezifische Bindungsaffinität aufweist, so dass sie auf wenigstens ein Gewebe eines biologischen Systems gerichtet ist.

5. Zusammensetzung nach Anspruch 1, wobei das zielgerichtete Mittel **dadurch** mit dem Radionuklid verknüpft ist, dass es an eine komplexierende Einheit gebunden ist, die das Radionuklid komplexiert.

6. Zusammensetzung nach Anspruch 5, wobei das an eine komplexierende Einheit gebundene zielgerichtete Mittel dargestellt wird durch die Formel:
A-CZ(B)-[C(R¹R²)]ₙ-X
worin A H, HOOC, H₂NOC, (Peptid, Oligonucleotid, Antikörper oder kleine organische Verbindung)-NHOC, (Peptid, Oligonucleotid, Antikörper oder kleine organische Verbindung)-OOC oder R⁴ ist; B H, SH oder -NHR³, -N(R³)-(Peptid, Oligonucleotid, Antikörper oder kleine organische Verbindung) oder R⁴ ist; X SH oder -NHR³, -N(R³)-(Peptid, Oligonucleotid, Antikörper oder kleine organische Verbindung) oder R⁴ ist; R¹, R², R³ und R⁴ unabhängig voneinander H oder gerad- oder verzweigtkettiges oder cyclisches Niederalkyl sind; n 0, 1 oder 2 ist; mit der Maßgabe, dass: (a) wenn B -NHR³ oder -N(R³)-(Peptid, Oligonucleotid, Antikörper oder kleine organische Verbindung) ist, X SH ist und n 1 oder 2 ist; (b) wenn X -NHR³ oder -N(R³)-(Peptid, Oligonucleotid, Antikörper oder kleine organische Verbindung) ist, B SH ist und n 1 oder 2 ist; (c) wenn B H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist, X SH ist und n 0 oder 1 ist; (d) wenn A H oder R⁴ ist und wenn B SH ist, X -NHR³ oder -N(R³)-(Peptid, Oligonucleotid, Antikörper oder kleine organische Verbindung) ist, und wenn X SH ist, B -NHR³ oder -N(R³)-(Peptid, Oligonucleotid, Antikörper oder kleine organische Verbindung) ist; (e) wenn X H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist; (f) wenn Z Methyl ist, X Methyl ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist und n 0 ist; und (g) wenn Z SH ist und X SH ist, n nicht 0 ist; wobei die Thiol-Einheit in der reduzierten Form vorliegt und die komplexierende Gruppe covalent an das Peptid, Oligonucleotid, den Antikörper oder die kleine organische Verbindung gebunden werden kann.

7. Zusammensetzung nach Anspruch 4, wobei das zielgerichtete Mittel ein Somatostatin-Rezeptor bindendes Peptid ist.

8. Zusammensetzung nach Anspruch 7, wobei das Somatostatin-Rezeptor bindende Peptid Depreotid oder P2045 ist.

9. Zusammensetzung nach Anspruch 1, wobei das Radionuklid Tc-99m ist.

10. Verfahren zur Stabilisierung einer Zusammensetzung, die ein Radionuklid umfasst, das mit einem zielgerichteten Mittel verknüpft ist, um das Auftreten eines Abbaus des Radionuklids zu verhindern oder zu verringern, wobei das Verfahren die Bereitstellung von Iodid-Ionen in der Zusammensetzung umfasst.

11. Verfahren nach Anspruch 10, wobei die Iodid-Ionen mit Hilfe eines Iodid-Salzes in der Zusammensetzung bereitgestellt werden.

12. Verfahren nach Anspruch 10, wobei die Iodid-Ionen mit Hilfe eines Alkalimetalliodid-Salzes in der Zusammensetzung bereitgestellt werden.

13. Verfahren nach Anspruch 10, wobei das zielgerichtete Mittel ein Peptid, Oligonucleotid, Antikörper, eine peptidomimetische oder kleine organische Verbindung ist, die spezifische Bindungsaffinität aufweist, so dass sie auf wenigstens ein Gewebe eines biologischen Systems gerichtet ist.

14. Verfahren nach Anspruch 10, wobei das zielgerichtete Mittel **dadurch** mit dem Radionuklid verknüpft ist, dass es an eine komplexierende Einheit gebunden ist, die das Radionuklid komplexiert.

15. Verfahren nach Anspruch 14, wobei das an eine komplexierende Einheit gebundene zielgerichtete Mittel dargestellt wird durch die Formel:
A-CZ(B)-[C(R¹R²)]ₙ-X
worin A H, HOOC, H₂NOC, (Peptid, Oligonucleotid, Antikörper oder kleine organische Verbindung)-NHOC, (Peptid, Oligonucleotid, Antikörper oder kleine organische Verbindung)-OOC oder R⁴ ist; B H, SH oder -NHR³, -N(R³)-(Peptid, Oligonucleotid, Antikörper oder kleine organische Verbindung) oder R⁴ ist; X SH oder -NHR³, -N(R³)-(Peptid, Oligonucleotid, Antikörper oder kleine organische Verbindung) oder R⁴ ist; R¹, R², R³ und R⁴ unabhängig voneinander H oder gerad- oder verzweigtkettiges oder cyclisches Niederalkyl sind; n 0, 1 oder 2 ist; mit der Maßgabe, dass: (a) wenn B -NHR³ oder -N(R³)-(Peptid, Oligonucleotid, Antikörper oder kleine organische Verbindung) ist, X SH ist und n 1 oder 2 ist; (b) wenn X -NHR³ oder -N(R³)-(Peptid, Oligonucleotid, Antikörper oder kleine organische Verbindung) ist, B SH ist und n 1 oder 2 ist; (c) wenn B H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist, X SH ist und n 0 oder 1 ist; (d) wenn A H oder R⁴ ist und wenn B SH ist, X -NHR³ oder -N(R³)-(Peptid, Oligonucleotid, Antikörper oder kleine organische Verbindung) ist, und wenn X SH ist, B -NHR³ oder -N(R³)-(Peptid, Oligonucleotid, Antikörper oder kleine organische Verbindung) ist; (e) wenn X H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist; (f) wenn Z Methyl ist, X Methyl ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist und n 0 ist; und (g) wenn Z SH ist und X SH ist, n nicht 0 ist; wobei die Thiol-Einheit in der reduzierten Form vorliegt und die komplexierende Gruppe covalent an das Peptid, Oligonucleotid, den Antikörper oder die kleine organische Verbindung gebunden werden kann.

16. Verfahren nach Anspruch 10, wobei das zielgerichtete Mittel ein Somatostatin-Rezeptor bindendes Peptid ist.

17. Verfahren nach Anspruch 16, wobei das Somatostatin-Rezeptor bindende Peptid Depreotid oder P2045 ist.

18. Verfahren nach Anspruch 10, wobei das Radionuklid Tc-99m ist.

19. Verfahren nach Anspruch 13, wobei das biologische System der Körper eines Säugers ist.

20. Verfahren nach Anspruch 19, des Weiteren umfassend die Verabreichung des Komplexes an den Körper eines Säugers und die Durchführung einer bildgebenden Szintigraphie am Körper des Säugers.

21. Kit, umfassend:
(a) ein zielgerichtetes Mittel, das mit einem Radionuklid verknüpft ist,
(b) Iodid-Ionen oder eine Verbindung, die Iodid-Ionen freisetzt oder erzeugt, wobei die Iodid-Ionen den Abbau des Radionuklids aufgrund von Radiolyse oder freien Ionen verhindern oder verringern, und
(c) Komponenten zur Erzeugung eines Radionuklids, das mit dem zielgerichteten Mittel verknüpft ist,
wobei das Kit zwei oder drei Kammern aufweist, (c) in einer von (a) oder (b) getrennten Kammer enthalten ist und (a) und (b) in der gleichen oder in verschiedenen Kammern sein können.

22. Kit nach Anspruch 21, wobei die Iodid-Ionen mit Hilfe eines Iodid-Salzes bereitgestellt werden.

23. Kit nach Anspruch 21, wobei die Iodid-Ionen mit Hilfe eines Alkalimetalliodid-Salzes bereitgestellt werden.

24. Kit nach Anspruch 21, wobei das zielgerichtete Mittel ein Peptid, Oligonucleotid, Antikörper, eine peptidomimetische oder kleine organische Verbindung ist, die spezifische Bindungsaffinität aufweist, so dass sie auf wenigstens ein Gewebe eines biologischen Systems gerichtet ist.

25. Kit nach Anspruch 21, wobei das zielgerichtete Mittel **dadurch** mit dem Radionuklid verknüpft werden kann, dass es an eine komplexierende Einheit gebunden werden kann, die das Radionuklid komplexiert.

26. Kit nach Anspruch 25, wobei das an eine komplexierende Einheit gebundene zielgerichtete Mittel dargestellt wird durch die Formel:
A-CZ(B)-[C(R¹R²)]ₙ-X
worin A H, HOOC, H₂NOC, (Peptid, Oligonucleotid, Antikörper oder kleine organische Verbindung)-NHOC, (Peptid, Oligonucleotid, Antikörper oder kleine organische Verbindung)-OOC oder R⁴ ist; B H, SH oder -NHR³, -N(R³)-(Peptid, Oligonucleotid, Antikörper oder kleine organische Verbindung) oder R⁴ ist; X SH oder -NHR³, -N(R³)-(Peptid, Oligonucleotid, Antikörper oder kleine organische Verbindung) oder R⁴ ist; R¹, R², R³ und R⁴ unabhängig voneinander H oder gerad- oder verzweigtkettiges oder cyclisches Niederalkyl sind; n 0, 1 oder 2 ist; mit der Maßgabe, dass: (a) wenn B -NHR³ oder -N(R³)-(Peptid, Oligonucleotid, Antikörper oder kleine organische Verbindung) ist, X SH ist und n 1 oder 2 ist; (b) wenn X -NHR³ oder -N(R³)-(Peptid, Oligonucleotid, Antikörper oder kleine organische Verbindung) ist, B SH ist und n 1 oder 2 ist; (c) wenn B H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist, X SH ist und n 0 oder 1 ist; (d) wenn A H oder R⁴ ist und wenn B SH ist, X -NHR³ oder -N(R³)-(Peptid, Oligonucleotid, Antikörper oder kleine organische Verbindung) ist, und wenn X SH ist, B -NHR³ oder -N(R³)-(Peptid, Oligonucleotid, Antikörper oder kleine organische Verbindung) ist; (e) wenn X H oder R⁴ ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist; (f) wenn Z Methyl ist, X Methyl ist, A HOOC, H₂NOC, (Peptid, Oligonucleotid, Antikörper oder kleine organische Verbindung)-NHOC, (Peptid, Oligonucleotid, Antikörper oder kleine organische Verbindung)-OOC ist und B SH ist und n 0 ist; und (g) wenn Z SH ist und X SH ist, n nicht 0 ist; wobei die Thiol-Einheit in der reduzierten Form vorliegt und die komplexierende Gruppe covalent an das Peptid, Oligonucleotid, den Antikörper oder die kleine organische Verbindung gebunden werden kann.

27. Kit nach Anspruch 21, wobei das zielgerichtete Mittel ein Somatostatin-Rezeptor bindendes Peptid ist.

28. Kit nach Anspruch 27, wobei das Somatostatin-Rezeptor bindende Peptid Depreotid oder P2045 ist.

29. Kit nach Anspruch 21, wobei das Radionuklid Tc-99m ist.

## Revendications

1. Composition comprenant :
- un radionucléide, facultativement sous forme de partie d'un composé ou d'un complexe, qui est associé à un agent de ciblage, et
- des ions iodure ou un composé qui libère ou génère des ions iodure, moyennant quoi les ions iodure aident à stabiliser la composition contre la dégradation en maintenant ainsi une haute pureté radiochimique de la composition.

2. Composition selon la revendication 1, dans laquelle les ions iodure sont fournis par un sel d'iodure dans la composition.

3. Composition selon la revendication 1, dans laquelle les ions iodure sont fournis par un sel d'iodure de métal alcalin dans la composition.

4. Composition selon la revendication 1, dans laquelle l'agent de ciblage est un peptide, un oligonucléotide, un anticorps, un composé peptidomimétique ou un petit composé organique qui a une affinité de liaison spécifique le faisant cibler au moins un tissu d'un système biologique.

5. Composition selon la revendication 1, dans laquelle l'agent de ciblage est associé au radionucléide en étant lié à un groupe complexant qui complexe le radionucléide.

6. Composition selon la revendication 5, dans laquelle l'agent de ciblage lié à un groupe complexant est représenté par la formule :
A--CZ(B)--[C(R¹R²)]ₙ--X
dans laquelle
A est H, HOOC, H₂NOC, (peptide, oligonucléotide, anticorps ou petit composé organique)-NHOC, (peptide, oligonucléotide, anticorps ou petit composé organique)-OOC ou R⁴,
B est H, SH ou --NHR³, --N(R³)-(peptide, oligonucléotide, anticorps ou petit composé organique) ou R⁴,
X est SH ou -NHR³, -N(R³)-(peptide, oligonucléotide, anticorps ou petit composé organique) ou R⁴,
R¹, R², R³ et R⁴ sont indépendamment H ou un alkyle inférieur à chaîne droite ou ramifiée ou cyclique,
n est 0,1 ou 2,
à condition que :
(a) lorsque B est -NHR³ ou -N(R³)-(peptide, oligonucléotide, anticorps ou petit composé organique), X est SH et n est 1 ou 2,
(b) lorsque X est -NHR³ ou -N(R³)-(peptide, oligonucléotide, anticorps ou petit composé organique), B est SH et n est 1 ou 2,
(c) lorsque B est H ou R⁴, A est HOOC, H₂NOC, (peptide, oligonucléotide, anticorps ou petit composé organique)-NHOC, (peptide, oligonucléotide, anticorps ou petit composé organique)-OOC, X est SH et n est 0 ou 1,
(d) lorsque A est H ou R⁴, et en outre, lorsque B est SH, X est -NHR³ ou -N(R³)-(peptide, oligonucléotide, anticorps ou petit composé organique) et lorsque X est SH, B est --NHR³ ou --N(R³)-(peptide, oligonucléotide, anticorps ou petit composé organique),
(e) lorsque X est H ou R⁴, A est HOOC, H₂NOC, (peptide, oligonucléotide, anticorps ou petit composé organique)-NHOC, (peptide, oligonucléotide, anticorps ou petit composé organique)-OOC et B est SH,
(f) lorsque Z est méthyl, X est méthyl, A est HOOC, H₂NOC, (peptide, oligonucléotide, anticorps ou petit composé organique)-NHOC, (peptide, oligonucléotide, anticorps ou petit composé organique)-OOC et B est SH et n est 0, et
(g) lorsque Z est SH et X est SH, n est différent de 0, et dans laquelle le groupe thiol est sous forme réduite et le groupe complexant est capable d'être lié de façon covalente au peptide, à l'oligonucléotide, l'anticorps ou au petit composé organique.

7. Composition selon la revendication 4, dans laquelle l'agent de ciblage est un peptide se liant à un récepteur de la somatostatine.

8. Composition selon la revendication 7, dans laquelle le peptide se liant à un récepteur de la somatostatine est du dépréotide ou du P2045.

9. Composition selon la revendication 1, dans laquelle le radionucléide est du Tc-99m.

10. Procédé pour stabiliser une composition comprenant un radionucléide associé à un agent de ciblage pour prévenir ou réduire l'apparition d'une dégradation du radionucléide, le procédé comprenant l'octroi d'ions iodure dans la composition.

11. Procédé selon la revendication 10, dans lequel les ions iodure sont fournis par un sel d'iodure dans la composition.

12. Procédé selon la revendication 10, dans lequel les ions iodure sont fournis par un sel d'iodure de métal alcalin dans la composition.

13. Procédé selon la revendication 10, dans lequel l'agent de ciblage est un peptide, un oligonucléotide, un anticorps, un composé peptidomimétique ou un petit composé organique qui a une affinité de liaison spécifique le faisant cibler au moins un tissu d'un système biologique.

14. Procédé selon la revendication 10, dans lequel l'agent de ciblage est associé à un radionucléide en étant lié à un groupe complexant qui complexe le radionucléide.

15. Procédé selon la revendication 14, dans lequel l'agent de ciblage lié à un groupe complexant est représenté par la formule :
A--CZ(B)--[C(R¹R²)]ₙ--X
dans laquelle
A est H, HOOC, H₂NOC, (peptide, oligonucléotide, anticorps ou petit composé organique)-NHOC, (peptide, oligonucléotide, anticorps ou petit composé organique)-OOC ou R⁴,
B est H, SH ou --NHR³, --N(R³)-(peptide, oligonucléotide, anticorps ou petit composé organique) ou R⁴,
X est SH ou -NHR³, -N(R³)-(peptide, oligonucléotide, anticorps ou petit composé organique) ou R⁴,
R¹, R², R³ et R⁴ sont indépendamment H ou un allyle inférieur à chaîne droite, ramifiée ou cyclique,
n est 0,1 ou 2,
à condition que :
(a) lorsque B est -NHR³ ou -N(R³)-(peptide, oligonucléotide, anticorps ou petit composé organique), X est SH et n est 1 ou 2,
(b) lorsque X est -NHR³ ou -N(R³)-(peptide, oligonucléotide, anticorps ou petit composé organique), B est SH et n est 1 ou 2,
(c) lorsque B est H ou R⁴, A est HOOC, H₂NOC, (peptide, oligonucléotide, anticorps ou petit composé organique)-NHOC, (peptide, oligonucléotide, anticorps ou petit composé organique)-OOC, X est SH et n est 0 ou 1,
(d) lorsque A est H ou R⁴, et en outre, lorsque B est SH, X est -NHR³ ou -N(R³)-(peptide, oligonucléotide, anticorps ou petit composé organique) et lorsque X est SH, B est --NHR³ ou --N(R³)-(peptide, oligonucléotide, anticorps ou petit composé organique),
(e) lorsque X est H ou R⁴, A est HOOC, H₂NOC, (peptide, oligonucléotide, anticorps ou petit composé organique)-NHOC, (peptide, oligonucléotide, anticorps ou petit composé organique)-OOC et B est SH,
(f) lorsque Z est méthyl, X est méthyl, A est HOOC, H₂NOC, (peptide, oligonucléotide, anticorps ou petit composé organique)-NHOC, (peptide, oligonucléotide, anticorps ou petit composé organique)-OOC et B est SH et n est 0, et
(g) lorsque Z est SH et X est SH, n est différent de 0,
et dans laquelle le groupe thiol est sous forme réduite et le groupe complexant est capable d'être lié de façon covalente au peptide, à l'oligonucléotide, l'anticorps ou au petit composé organique.

16. Procédé selon la revendication 10, dans lequel l'agent de ciblage est un peptide se liant à un récepteur de la somatostatine.

17. Procédé selon la revendication 16, dans lequel le peptide se liant à un récepteur de la somatostatine est du dépréotide ou du P2045.

18. Procédé selon la revendication 10, dans lequel le radionucléide est du Tc-99m.

19. Procédé selon la revendication 13, dans lequel le système biologique est un corps de mammifère.

20. Procédé selon la revendication 19, comprenant en plus l'administration du complexe à un corps de mammifère et la réalisation d'une imagerie scintigraphique du corps de mammifère.

21. Kit comprenant :
(a) un agent de ciblage associé à un radionucléide,
(b) des ions iodure ou un composé qui libère ou produit des ions iodure, lesquels ions iodure préviennent ou réduisent la dégradation du radionucléide due à la radiolyse ou aux ions libres, et
(c) des composants destinés à générer un radionucléide associé avec l'agent de ciblage,
dans lequel le kit a deux ou trois compartiments, (c) est contenu dans un compartiment séparé de (a) ou (b) et (a) et (b) peuvent être dans le même compartiment ou dans des compartiments différents.

22. Kit selon la revendication 21, dans lequel les ions iodure sont fournis par un sel d'iodure.

23. Kit selon la revendication 21, dans lequel les ions iodure sont fournis par un sel d'iodure de métal alcalin.

24. Kit selon la revendication 21, dans lequel l'agent de ciblage est un peptide, un oligonucléotide, un anticorps, composé peptidomimétique ou un petit composé organique qui a une affinité de liaison spécifique le faisant cibler au moins un tissu d'un système biologique.

25. Kit selon la revendication 21, dans lequel l'agent de ciblage est capable d'être associé avec le radionucléide en étant capable d'être lié à un groupe complexant qui complexe le radionucléide.

26. Kit selon la revendication 25, dans lequel l'agent de ciblage lié à un groupe complexant est représenté par la formule :
A--CZ(B)--[C(R¹R²)]ₙ--X
dans laquelle
A est H, HOOC, H₂NOC, (peptide, oligonucléotide, anticorps ou petit composé organique)-NHOC, (peptide, oligonucléotide, anticorps ou petit composé organique)-OOC ou R⁴,
B est H, SH ou --NHR³, --N(R³)-(peptide, oligonucléotide, anticorps ou petit composé organique) ou R⁴,
X est SH ou -NHR³, -N(R³)-(peptide, oligonucléotide, anticorps ou petit composé organique) ou R⁴,
R¹, R², R³ et R⁴ sont indépendamment l'un de l'autre H ou un allyle inférieur à chaîne droite ou ramifiée ou cyclique,
n est 0,1 ou 2,
à condition que :
(a) lorsque B est -NHR³ ou -N(R³)-(peptide, oligonucléotide, anticorps ou petit composé organique), X est SH et n est 1 ou 2,
(b) lorsque X est -NHR³ ou -N(R³)-(peptide, oligonucléotide, anticorps ou petit composé organique), B est SH et n est 1 ou 2,
(c) lorsque B est H ou R⁴, A est HOOC, H₂NOC, (peptide, oligonucléotide, anticorps ou petit composé organique)-NHOC, (peptide, oligonucléotide, anticorps ou petit composé organique)-OOC, X est SH et n est 0 ou 1,
(d) lorsque A est H ou R⁴, et en outre, lorsque B est SH, X est -NHR³ ou -N(R³)-(peptide, oligonucléotide, anticorps ou petit composé organique) et lorsque X est SH, B est --NHR³ ou --N(R³)-(peptide, oligonucléotide, anticorps ou petit composé organique),
(e) lorsque X est H ou R⁴, A est HOOC, H₂NOC, (peptide, oligonucléotide, anticorps ou petit composé organique)-NHOC, (peptide, oligonucléotide, anticorps ou petit composé organique)-OOC et B est SH,
(f) lorsque Z est méthyl, X est méthyl, A est HOOC, H₂NOC, (peptide, oligonucléotide, anticorps ou petit composé organique)-NHOC, (peptide, oligonucléotide, anticorps ou petit composé organique)-OOC et B est SH et n est 0, et
(g) lorsque Z est SH et X est SH, n est différent de 0,
et dans laquelle le groupe thiol est sous forme réduite et le groupe complexant peut être lié de façon covalente au peptide, à l'oligonucléotide, l'anticorps ou au petit composé organique.

27. Kit selon la revendication 21, dans lequel l'agent de ciblage est un peptide se liant à un récepteur de la somatostatine.

28. Kit selon la revendication 27, dans lequel le peptide se liant à un récepteur de la somatostatine est du dépréotide ou du P2045.

29. Kit selon la revendication 21, dans lequel le radionucléide est du Tc-99m.
